# EUROPEAN PATENT APPLICATION

(11) **EP 4 527 371 A1**
(43) Date of publication of application: **26.03.2025**
(21) Application number: 23806506.4
(22) Date of filing: 16.01.2023
(51) Int. Cl.: A61K 8/67, A61K 8/14, A61Q 19/08, A61Q 19/00

(54) **PREPARATION METHOD FOR AND USE OF INCLUSION OF RETINOL AND DERIVATIVE THEREOF**

(30) Priority: 19.05.2022 CN 202210557526
(71) Applicant: Shanghai Oli Enterprises Co., Ltd., Shanghai 201403 (CN); JIANGSU OJI BIOTECH CO., LTD., Suzhou, Jiangsu 215635 (CN); Shanghai Oli Bio-Tech., Ltd., Fengxian District, Shanghai 201403 (CN)
(72) Inventor: LI, Xiaohu, Shanghai 201403 (CN); TAO, Li, Shanghai 201403 (CN); CHENG, Weifeng, Shanghai 201403 (CN); FANG, Huijing, Shanghai 201403 (CN); HUANG, Chong, Shanghai 201403 (CN); ZHU, Liping, Shanghai 201403 (CN); ZHANG, Weiyang, Shanghai 201403 (CN); QIAN, Jia, Shanghai 201403 (CN)
(74) Representative: Mewburn Ellis LLP
(86) International application number: PCT/CN2023/072447
(87) International publication number: WO 2023/221544

(57) **Abstract**

The present invention provides a preparation method for and use of an inclusion oily stock solution of retinol and a derivative thereof. Specifically, the inclusion oily stock solution comprises the following components in 100 parts by weight: 0.2-15 parts by weight of the retinol and the derivative thereof, 0.5-10 parts by weight of a liposomal structural analog, 10-50 parts by weight of an emulsifier, and the balance of grease. The inclusion of the retinol and the derivative thereof provided by the present invention has the advantages of high stability, high solubility, low irritation, good anti-aging effect and the like. It can be widely applied to anti-aging skin-care products such as essences, emulsions, creams, and masks.

## Description

### FIELD OF THE INVENTION

The present invention relates to the field of cosmetic raw materials, specifically to the preparation method for and use of inclusion of retinol and derivatives thereof.

### BACKGROUND

Anti-aging skincare products have been a hot topic and one of the main demands of consumers since ancient times.

As biological organisms grow, develop, and mature, they gradually enter the aging stage. On the one hand, they are regulated by genes, and on the other hand, they are also affected by various harmful external factors, that is, the dual effects of endogenous and exogenous factors, thereby accelerating aging. Skin is the most intuitive external manifestation of the body, the aging of which is visible to the naked eye, and which is also an organ directly affected by exogenous factors. In recent years, cosmetics technology has rapid developed, and a large number of anti-aging ingredients and skincare products have emerged, such as anthocyanins, astaxanthin, superoxide dismutase, vitamin C, vitamin E, coenzyme Q10, metallothionein, retinol and derivatives thereof, etc.

Vitamin A is a fat soluble vitamin that was initially used to treat rough and keratinized skin. It is an essential vitamin for maintaining the health of skin epithelial tissue and an important factor affecting cell metabolism. In recent years, it has been favored by consumers as a proven effective anti-aging ingredient for the skin. However, the special physicochemical properties of retinol limit its application, for example, it is prone to deactivation and degradation under light, heat, and oxygen conditions, and can cause irritating problems such as skin redness, swelling, heating, and pain.

Retinol derivatives obtained via side chain modification of chemical structure can improve stability and reduce irritation to a certain extent, but the corresponding efficacy will be weakened to varying degrees. The retinol family is vast, with various isomers or derivatives, such as retinol, retinal, retinoic acid, retinol palmitate, hydrogenated retinol, retinol acetate, retinol linoleate, retinol propionate, retinol retinoate, hydroxypinacolone retinoate, etc.

Hydroxypinacolone retinoate, abbreviated as HPR, belong to the retinoids family. The structural formula is as follows:

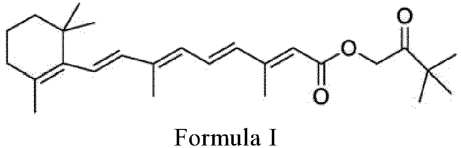

Hydroxypinacolone retinoate (HPR) is a retinol derivative modified with hydroxypinacolone, which has direct effects, low molecular weight, easier to transdermal absorption, low irritation, and high stability. Compared with other types of retinoids, HPR has obvious advantages. It was reported that multiple retinoids were compared in anti-aging and irritant effects using *in vitro* models of human skin, HPR has a stronger effect on gene transcription than retinol, retinal, and retinol palmitate, and its cytotoxicity is even lower at a concentration of 10 times. Compared to the untreated control, HPR is considered an alternative to retinoic acid for skin anti-aging with fewer skin side effects based on these results.

Although HPR has higher stability and better anti-aging effects compared to other retinoids, it has been found in practical applications that the application scenarios are complex and varied, and the external environment still poses certain challenges to its stability, inevitably leading to degradation.

Therefore, there is an urgent need in this field for a retinol product with excellent stability, high transparency, high content of active ingredient, and excellent efficacy.

### SUMMARY OF THE INVENTION

The purpose of the invention is to provide a preparation method for and use of an inclusion oily stock solution of retinol and a derivative thereof with excellent stability, high transparency, high content of active ingredient, and excellent efficacy.

The first aspect of the present invention provides an inclusion oily stock solution of retinol and a derivative thereof, comprising the following components in 100 parts by weight:
0.2-15 parts by weight of the retinol and the derivative thereof;
0.5-10 parts by weight of a liposomal structural analog;
10-50 parts by weight of an emulsifier; and
the balance of grease.

In another preferred embodiment, the oily stock solution comprises the following components in 100 parts by weight:
0.5-15 parts by weight of the retinol and the derivative thereof, such as 1, 2, 3, 5, 10, and 12;
0.5-10 parts by weight of a liposomal structural analog, such as 1, 2, 3, 5, 7, and 9;
15-40 parts by weight of an emulsifier, such as 18, 20, 25, and 30; and
the balance of grease.

In another preferred embodiment, the oily stock solution comprises the following components in 100 parts by weight:
0.5-12 parts by weight of the retinol and the derivative thereof;
1-10 parts by weight of a liposomal structural analog;
20-30 parts by weight of an emulsifier; and
the balance of grease.

In another preferred embodiment, the oily stock solution comprises the following components in 100 wt%:
0.2-15 wt% of the retinol and the derivative thereof, preferably 0.5-15wt%, more preferably 0.5-12wt%, such as 1wt%, 2wt%, 3wt%, 5wt%, 10wt%, and 12wt%;
0.5-10 wt% of a liposomal structural analog, preferably 0.5-10wt%, and more preferably 1-10wt%, such as 1wt%, 2wt%, 3wt%, 5wt%, 7wt%, and 9wt%;
10-50 wt% of an emulsifier, preferably 15-40wt%, more preferably 20-30wt%, such as 18wt%, 20wt%, 25wt%, and 30wt%; and
the balance of grease, based on the total mass of the oily stock solution.

In another preferred embodiment, the retinol and the derivative thereof are selected from the group consisting of retinol, retinal, retinoic acid, retinol palmitate, hydrogenated retinol, retinol acetate, retinol linoleate, retinol propionate, retinol retinoate, hydroxypinacolone retinoate, and combinations thereof.

In another preferred embodiment, the retinol derivative is hydroxypinacolone retinoate.

In another preferred embodiment, the retinol derivative is retinol palmitate.

In another preferred embodiment, the retinol derivative is represented by the following formula I:

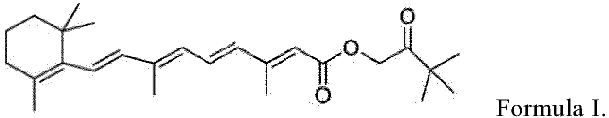

In another preferred embodiment, the retinol derivative is represented by the following formula II:

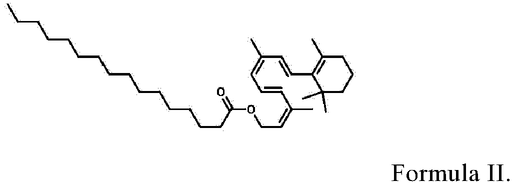

In another preferred embodiment, the liposomal structural analog is a polymer comprising:
(a) backbone unit A; and
(b) hydrophilic group L1 and optionally (c) hydrophobic group L2 located on the side chain or end of the polymer.

In another preferred embodiment, the polymer is a homopolymer, a copolymer, wherein the copolymer comprises a random copolymer, a block copolymer, and combinations thereof.

In another preferred embodiment, the liposomal structural analog is a phosphorylcholine polymer.

In another preferred embodiment, the hydrophilic group L1 is represented by formula II: 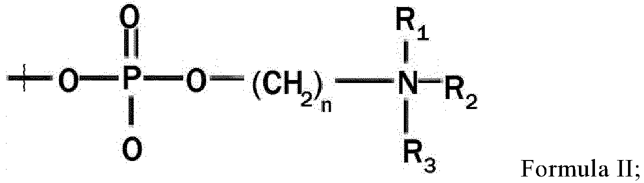
wherein, R₁, R₂, and R₃ are each independently selected from H, C₁-C₄ alkyl, C₂-C₄ alkenyl, and C₃-C₄ cycloalkyl;
n is an integer from 1 to 50 (e.g. 1, 2, 3, 4, 5, 10, or 15).

In another preferred embodiment, R₁, R₂ and R₃ are each independently selected from H, C₁-C₃ alkyl, and C₃-C₄ cycloalkyl, preferably methyl, ethyl, n-propyl, isopropyl, or cyclopropylalkyl.

In another preferred embodiment, R₁, R₂, and R₃ are all methyl.

In another preferred embodiment, the hydrophilic group L1 is located on the side chain, end or branch of the polymer.

In another preferred embodiment, the hydrophobic group L2 is a C₅₋₃₀ fatty chain structure.

In another preferred embodiment, the backbone unit A is selected from the group consisting of:
A1) 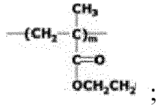 A2) 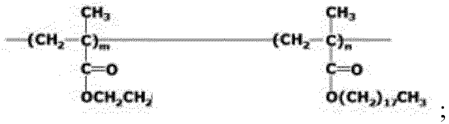
A3) 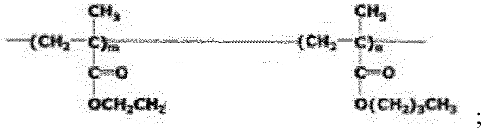
A4) 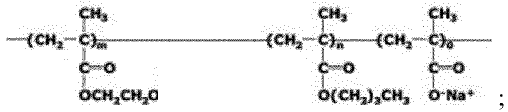 and
A5) combinations of A1-A4;
wherein, in A1 to A5, each m, n, and o is independently selected from an integer ranging from 1 to 1000 (2, 3, 5, 8, 10, 15, 20, 50, 100, 200, 300, 500, or 1000). In another preferred embodiment, in A1 to A5, the hydrophilic group L1 is connected to the backbone unit A via - CO-O-CH₂-CH₂-, -CO-O-CH₂-CH₂-O-, and/or -CO-O-(CH₂)ₚ-CH₂-, wherein p is an integer from 2 to 17.

In another preferred embodiment, the backbone unit A is: 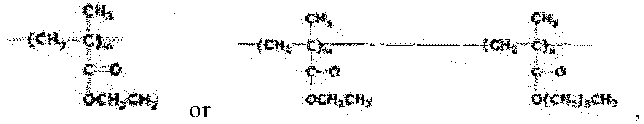 wherein, in the backbone unit A, each m and n is independently selected from an integer from 1 to 20.

In another preferred embodiment, the liposomal structural analog is selected from the group consisting of 2-methylacryloyloxyethylphosphocholine homopolymer, copolymers of 2-methylacryloyloxyethylphosphocholine and other hydrophilic and/or hydrophobic monomers and combinations thereof, polyphosphorylcholine glycol acrylate, polyphosphorylcholine *n-*butyl methacrylate, and combinations thereof.

In another preferred embodiment, the molecular weight of the liposomal structural analog is between 10,000 and 3 million, more preferably between 50,000 and 2 million, such as 100,000, 200,000, 300,000, 500,000, 700,000, 1 million or 1.5 million.

In another preferred embodiment, the retinol and the derivative thereof are encapsulated by the liposomal structural analog to form the inclusion.

In another preferred embodiment, the grease is selected from the group consisting of dioctyl carbonate, octyldodecanol isostearate, isopropyl palmitate, isooctyl palmitate, isononyl isononanoate, white oil, caprylic/capric triglyceride, octyldodecanol, cocinic acid, lauryl alcohol, isopropyl myristate, shea butter oil, isooctyl 12-hydroxystearate, diisostearyl malate, squalane, jojoba oil, soybean oil, olive oil, sweet almond oil, avocado oil, wheat germ oil, and combinations thereof.

In another preferred embodiment, the emulsifier is selected from the group consisting of polyglycerol esters, dehydrated sorbitol fatty acid esters, sorbitol cocoate, sucrose fatty acid esters, alkyl glycosides, hydrogenated soft phospholipids, fatty alcohol ethoxylates, alkylphenol ethoxylates, polyoxyethylene fatty acid, polyethers, caprylic/capric glycerides, and combinations thereof.

The second aspect of the present invention provides a method for preparing the inclusion oily stock solution according to the first aspect of the present invention, comprising steps of:
(1) providing a liposomal structural analog and an emulsifier, and mixing them to obtain an oil phase mixture;
(2) subjecting the oil phase mixture in step (1) to high-temperature homogenization and stirring to form a mixture;
(3) cooling down subsequently;
(4) adding retinol and the derivative thereof, stirring and homogenizing to form an inclusion mixture; and
(5) adding grease and stirring thoroughly to obtain the inclusion oily stock solution.

In another preferred embodiment, in step (2), the oil phase mixture is stirred at 80-95 °C.

In another preferred embodiment, in step (3), the temperature is cooled to 20-45 °C, preferably 20-40 °C.

In another preferred embodiment, the stirring is high-speed stirring, preferably at 500-700 rpm.

The third aspect of the present invention provides a cosmetic composition comprising the inclusion oily stock solution according to the first aspect of the present invention.

In another preferred embodiment, the cosmetic composition is an oil-based formula cosmetic.

In another preferred embodiment, the dosage form of the cosmetic composition is a solid dosage form, a semi-solid dosage form, or a liquid dosage form.

In another preferred embodiment, the cosmetic composition is an anti-aging skincare product.

In another preferred embodiment, the cosmetic composition includes solution, gel, cream, lotion, ointment, cream, cake, powder or paste.

In another preferred embodiment, the cosmetic composition includes essence liquid, lotion, cream or facial mask.

In another preferred embodiment, the cosmetic composition further comprises cosmetic acceptable excipients.

The fourth aspect of the present invention provides a use of the inclusion oily stock solution according to the first aspect of the present invention for preparing an oil-based formula cosmetic composition, wherein the cosmetic composition is an anti-aging cosmetic.

The fifth aspect of the present invention provides an inclusion of retinol and the derivative thereof, comprising the following components in parts by weight:
0.2-15 parts by weight of the retinol and the derivative thereof;
0.5-10 parts by weight of a liposomal structural analog; and
10-50 parts by weight of an emulsifier;
wherein, the retinol and the derivative thereof are encapsulated by liposomal structural analog, thereby forming an inclusion.

In another preferred embodiment, the inclusion comprises the following components in parts by weight:
0.5-15 parts by weight of the retinol and the derivative thereof, such as 1, 2, 3, 5, 8, 10, and 12;
0.5-10 parts by weight of a liposomal structural analog, such as 1, 2, 3, 5, 7, and 9; and
15-40 parts by weight of an emulsifier, such as 18, 20, 25, and 30.

In another preferred embodiment, the inclusion comprises the following components:
0.2-15 wt% of the retinol and the derivative thereof, preferably 0.5-15wt%, more preferably 0.5-12wt%, such as 1wt%, 2wt%, 3wt%, 5wt%, 10wt%, and 12wt%;
0.5-10 wt% of a liposomal structural analog, preferably 0.5-10wt%, and more preferably 1-10wt%, such as 1wt%, 2wt%, 3wt%, 5wt%, 7wt%, and 9wt%; and
10-50 wt% of an emulsifier, preferably 15-40wt%, more preferably 20-30wt%, such as 18wt%, 20wt%, 25wt%, and 30wt%, based on the total mass of the inclusion.

In another preferred embodiment, the retinol and the derivative thereof, liposomal structural analog, and emulsifier are each independently as described in the first aspect of the present invention.

In another preferred embodiment, the size of the inclusion is 70-150 nm, preferably 70-120 nm.

The sixth aspect of the present invention provides a method for preparing an inclusion according to the fifth aspect of the present invention, comprising the following steps:
(1) providing a liposomal structural analog and an emulsifier, and mixing them to obtain an oil phase mixture;
(2) subjecting the oil phase mixture in step (1) to high-temperature homogenization and stirring to form a mixture, then cooling down; and
(3) adding the retinol and the derivative thereof, stirring and homogenizing to form the inclusion.

In another preferred embodiment, in step (2), the oil phase mixture is homogenized and stirred at 80-95 °C.

In another preferred embodiment, in step (2), the temperature is cooled to 20-45 °C, preferably 20-40 °C.

In another preferred embodiment, the stirring is high-speed stirring, preferably at 500-700 rpm.

It should be understood that within the scope of the present invention, the above-described technical features of the present invention and the technical features described in detail below (e.g., examples) may be combined with each other to constitute a new or preferred technical solution. Limited by space, it will not be repeated here.

### DESCRIPTION OF THE DRAWINGS

Figure 1 shows the optical photograph of HPR inclusion oily stock solution No.1 prepared in Example 1.
Figure 2 shows the optical photograph of retinol palmitate inclusion oily stock solution No.6 prepared in Example 6.
Figure 3 shows the optical photograph of HPR mixture No.7 prepared in Comparative Example 1.
Figure 4 shows the optical photograph of HPR mixture No.8 prepared in Comparative Example 2.
Figure 5 shows the transmission electron microscopy image of the morphology of the inclusion.
Figure 6 shows high-definition VISA images of volunteers using eye cream prepared from HPR inclusion oily stock solution No.1 for a period of time.

### DETAILED DESCRIPTION OF THE INVENTION

After extensive and in-depth research, the inventors unexpectedly developed for the first time an inclusion oily stock solution with excellent stability, low irritation, and high content of active ingredient. Specifically, the inventors greatly improved the stability of retinol and the analogues thereof by adding specific liposomal structural analog, and due to the reduced irritation and increased solubility caused by encapsulation, the stability of the product and the content of active ingredient in the product were greatly improved, the quality and anti-aging effect of cosmetics were enhanced. The present invention has been completed on this basis.

### Terms

Unless otherwise defined, the meanings of all technical and scientific terms used herein are the same as those generally understood by those skilled in the art to which the present invention belongs.

As used herein, the terms "include," "comprise" and "contain" are used interchangeably to include not only closed definitions, but also semi-closed, and open definitions. In other words, the term includes "consist of" and "substantially consist of".

### Active Ingredient

The active ingredient of the present invention refers to the retinol and the derivative thereof.

Retinol and the derivative thereof belong to vitamin A, both of which are fat soluble compounds. The biggest limitation of this type of compounds in terms of application is highly degradable and the presence of certain irritants.

The active ingredient of the present invention is selected from the group consisting of retinol, retinal, retinoic acid, retinol palmitate, hydrogenated retinol, retinol acetate, retinol linoleate, retinol propionate, retinol retinoate, hydroxypinacolone retinoate, and combinations thereof.

In another preferred embodiment, the active ingredient is hydroxypinacolone retinoate or retinol palmitate.

Hydroxypinaclone retinate, abbreviated as HPR, is a derivative of retinol with the structural formula shown in formula I:

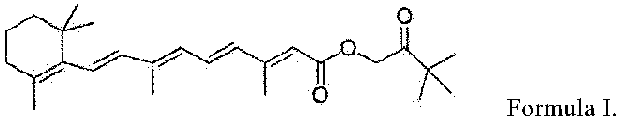

The structural formula of retinol palmitate is shown in Formula II,

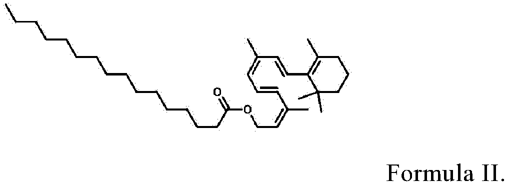

### Liposomal structural analog

The liposomal structural analog of the present invention is an amphiphilic polymer similar to liposomes. The iposomal structural analog of the present invention comprises: (a) backbone unit A; (b) hydrophilic group L1 and optionally (c) hydrophobic group L2 located on the side chain or end of the polymer.

The hydrophilic group contains a structure shown in formula II (preferably as side chain group or branched structure): 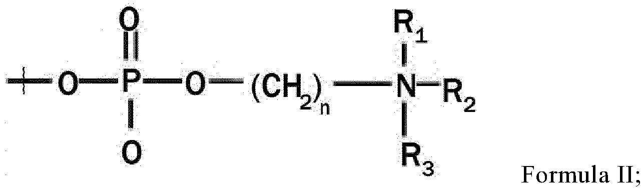
wherein, R₁, R₂, and R₃ are each independently selected from H, C₁-C₄ alkyl, and C₃-C₄ cycloalkyl;
n is 1, 2, 3, or 4.

Preferably, R₁, R₂, and R₃ are each independently selected from H, methyl, and ethyl.

Preferably, R₁, R₂, and R₃ are all CH₃.

Preferably, n is 2 or 3.

In the present invention, the hydrophilic group L1 can be directly connected to the polymer backbone (such as terminated-linked to the polymer), or indirectly connected to the backbone via a linking group (such as a divalent linking group, or a divalent linking group).

The hydrophobic group L2 comprises a C₅₋₃₀ fatty chain structure.

In the present invention, the hydrophobic group L2 can be located on the side chain or at the end.

The backbone unit of the liposomal structural analog of the present invention is:

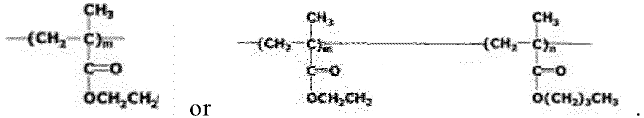

In another preferred embodiment, the molecular weight of the liposome structural analog is not particularly limited, the weight average molecular or number average molecular weight of which is preferably 10,000 to 3 million, more preferably 50,000 to 2 million.

The liposome structural analog described in the present invention is a phosphorylcholine polymer, which refers to a polymer containing phosphorylcholine group. The polymer can be a homopolymer or a copolymer, including a random copolymer, a block copolymer, and combinations thereof. The copolymer can be a binary, ternary, or multicomponent copolymer.

Preferably, the liposome structural analog of the present invention is a copolymer formed by copolymerizing hydrophilic monomers (monomers containing phosphorylcholine groups) with hydrophobic monomers (monomers without phosphorylcholine groups).

More preferably, the liposome structural analog of the present invention is polyquaternium-51.

The inclusion oily stock solution of the present invention (1) utilizes hydrophobic chain groups (or monomers) and hydrophilic groups (or monomers) in the structure of liposome structural analogs to form a dense wall material, encapsulating retinol and the derivative thereof to form a molecular level active ingredient inclusion, thereby significantly improving stability; (2) utilizes liposome structural analogs and cell-membrane-like structures to reduce the irritation of the product and enhance the transdermal absorption effect; (3) utilizes the amphiphilicity of liposome structural analogs to improve the solubility, the inclusion is fully dispersed in oils, resulting in a high content of active ingredients dissolved in the solution, finally obtaining an oily stock solution with high stability, high content, low irritation, and high transparency, significantly increasing content of its active ingredient and permeability, enhancing its anti-aging and other effects or efficacy.

### The inclusion oily stock solution of the present invention

As used herein, the "inclusion oily stock solution of the present invention" and "oily stock solution" can be used interchangeably, which refer to the oily stock solution obtained by encapsulating active ingredient with liposomal structural analogs.

The oily stock solution of the present invention comprises the following components: 0.5-15% of retinol and the derivative thereof, 0.5-10% of a liposomal structural analog, 1-30% of an emulsifier, and the balance of grease, based on the total weight of the oily stock solution.

In another preferred embodiment, the oily stock solution contains the surplus of grease.

The retinol and the derivative thereof in the inclusion oily stock solution of the present invention may refer only to retinol, or only to one or more derivatives of retinol, or a mixture of two or more retinol and the derivative thereof.

The inclusion oily stock solution of the invention is not only conducive to the application of retinol and the derivative thereof in anti-aging skin care products such as an essence solution, a lotion, a cream, a facial mask, etc., but also can promote the penetration on the skin, improve the stability and anti-aging effect, and solve the problems of poor stability, high irritation, etc. in the prior art.

The present invention surprisingly found that using liposomal structural analog for encapsulation modification of HPR and retinol, leading to the obtained inclusion has higher stability, high content, and the irritability was further reduced through cell phospholipids, the transdermal absorption was better, and with significant effects.

It is worth noting that the present invention is not limited to the modification of HPR and retinol, but provides a modifiable platform that can modify various retinol and the derivatives thereof, greatly expanding the application scope of the retinols family, improving product stability, and having broad application prospects.

The present invention also provides a method for preparing the inclusion oily stock solution of the present invention, the method comprises the steps of:
(1) providing a liposomal structural analog and an emulsifier, and mixing them in a certain proportion to form an oil phase mixture;
(2) subjecting the oil phase mixture in step (1) to homogenization and stirring (80-95 °C) to form a mixture;
(3) cooling down (such as cooling to 20-45 °C, preferably 20-40 °C) subsequently;
(4) adding retinol and the derivative thereof, stirring and homogenizing to form an inclusion mixture; and
(5) adding grease and stirring thoroughly to obtain the inclusion oily stock solution.

### Cosmetic composition and use

The composition or product of the present invention includes the inclusion oily stock solution of the present invention.

The inclusion oily stock solution of the present invention can be combined with cosmetic acceptable carriers or excipients to form a cosmetic composition.

To the extent that the effects of the present invention are not hindered, other ingredients commonly used in cosmetics may be added into the cosmetics of the present invention, such as film formers, oil-soluble gelling agents, organically modified clay minerals, resins, moisturizers, preservatives, antibacterial agents, flavors, salts, antioxidants, pH adjusters, chelating agents, cooling agents, anti-inflammatory agents, ingredients for skin beautification (whitening agents, cytoactive agents, skin roughness improving agents, blood circulation promoters, skin firming agents, anti-lipid leakage agents, etc.), vitamins, amino acids, nucleic acids, hormones, inclusion compounds, etc.

The oil-soluble gelling agent is selected from metal soaps such as aluminum stearate, magnesium stearate, and zinc myristate; amino acid derivatives such as N-lauroyl-L-glutamic acid, α,γ-di-*n*-butylamine; cyclodextrin fatty acid esters such as cyclodextrin palmitate, cyclodextrin stearate, and cyclodextrin 2-ethylhexanoic acid palmitate; sucrose fatty acid esters such as sucrose palmitate and sucrose stearate; benzylidene derivatives of sorbitol such as monobenzylidene sorbitol and dibenzylidene sorbitol; gelling agent of organically modified clay minerals such as dimethylbenzyldodecylammonium montmorillonite clay and dimethyldioctadecylammonium montmorillonite clay. One, two or more types of agents may be used as required.

Humectants include glycerin, sorbitol, propylene glycol, dipropylene glycol, 1,3-butanediol, glucose, xylitol, maltitol, polyethylene glycol, hyaluronic acid, chondroitin sulfate, pyrrolidone carboxylate, polyoxyethylene methyl glucoside, polyoxypropylene methyl glucoside, etc.

Antibacterial preservatives include alkyl *p*-hydroxybenzoate, benzoic acid, sodium benzoate, sorbic acid, potassium sorbate, phenoxyethanol, etc.. Antibacterial agents include benzoic acid, salicylic acid, carbolic acid, sorbic acid, alkyl *p*-hydroxybenzoate, *p*-chloro-*m-*cresol, hexachlorophenol, benzalkonium chloride, chlorhexidine chloride, trichloro-N-carbanilide, triclosan, photosensitizer, phenoxyethanol, etc.

Antioxidants include tocopherol, butylhydroxyanisole, dibutylhydroxytoluene, phytic acid, etc. pH regulators include lactic acid, citric acid, glycolic acid, succinic acid, tartaric acid, dl-malic acid, potassium carbonate, sodium bicarbonate, ammonium bicarbonate, etc.. Chelating agents include alanine, sodium ethylenediaminetetraacetate, sodium polyphosphate, sodium metaphosphate, phosphoric acid, etc.. Cooling agents include L-menthol, camphor, etc.. Anti-inflammatory agents include allantoin, glycyrrhetinic acid, glycyrrhizic acid, tranexamic acid, azulene, etc.

Ingredients for skin beautification include whitening agents such as placenta extract, arbutin, glutathione and saxifrage extract; cytoactive agents such as royal jelly, photoreceptor, cholesterol derivatives, calf blood extract; skin roughness improving agents; blood circulation promoters such as valeramide pelargonate, benzyl nicotinate, β-butoxyethyl nicotinate, capsaicin, gingerone, cantharidin tincture, ichthyol, caffeine, tannic acid, α-borneol, tocopherol nicotinate, inositol hexanicotinate, cyclomandelate, cinnarizine, tolazoline, acetylcholine, verapamil, stephane and γ-oryzanol; skin firming agents such as zinc oxide, tannic acid; anti-lipid leakage agents such as sulfur. Vitamins include vitamin A such as rosin oil, rosin acetate, rosin palmitate; vitamin B2 such as riboflavin, riboflavin butyrate and flavin adenine nucleotides; vitamin B6 such as pyridoxine hydrochloride, pyridoxine dicaprylate, pyridoxine tripalmitate, vitamin B such as vitamin B12 and its derivatives, vitamin B15 and its derivatives; vitamin C such as L-ascorbic acid, L-ascorbyl dipalmitate, sodium L-ascorbate -2-sulfate and dipotassium L-ascorbate phosphate; vitamin D such as ergocalciferol and cholecalciferol; vitamin E such as α-tocopherol, β-tocopherol, γ-tocopherol, dl-α-tocopherol acetate, dl-α-tocopherol nicotinic acid, dl-α-tocopherol succinate; vitamin H; vitamin P; niacin such as nicotinic acid, benzyl nicotinate, niacinamide; pantothenic acid such as calcium pantothenate, D-panthenol, pantothen ethyl ether and acetyl pantothen ethyl ether; biotin and the like.

Amino acids include glycine, valine, leucine, isoleucine, serine, threonine, phenylalanine, arginine, lysine, aspartic acid, glutamic acid, cystine, cysteine, methionine and tryptophan. Nucleic acids include deoxyribonucleic acid and the like, and hormones include estradiol, vinyl estradiol and the like.

There is no particular limitation on the form of the product, and it may be liquid, emulsion, cream, solid, paste, gel, powder, spray, and the like.

Preferred examples of the cosmetic composition of the invention include (but are not limited to): essences, lotions, ointments, creams, facial masks, etc.

### The main advantages of the present invention include:

(a) The inclusion oily stock solution of the present invention utilizes liposomal structural analog to form an inclusion of retinol or the derivative thereof, significantly improving the stability of retinol or the derivative thereof (the content is reduced by less than 0.2% within 30 days of light exposure), especially significantly enhancing the stability of retinol itself (which is less stable than the derivatives thereof).
(b) In the oily stock solution of the present invention, the irritability of the active ingredient is reduced after modification with liposomal structural analog.
(c) In the oily stock solution of the present invention, the particle size of the inclusion of liposomal structural analog and retinol and the derivative thereof is at the molecular level, thereby the penetration of the active ingredient in cosmetics made with the oily stock solution of the present invention is strong, thereby enhancing the anti-aging effect of the cosmetics.
(d) The solubility of retinol derivatives in the oily stock solution of the present invention is increased, resulting in an increase in the content of active ingredient (up to 12%) in cosmetic compositions made from the oily stock solution of the present invention.
(e) The inclusion oily stock solution of the present invention has good transparency and is clear and transparent.
(f) The inclusion oily stock solution of the present invention has a wide range of applications, the oily stock solution of the present invention can be directly used in essences, lotions, creams, facial masks and other cosmetics.

The present invention will be further explained below in combination with specific examples. It should be understood that these examples are only used to illustrate the present invention and not to limit the scope of the present invention. In the following examples, the experimental methods without specific conditions are usually in accordance with conventional conditions or the conditions recommended by the manufacturer. Unless otherwise specified, percentages and parts are calculated by weight.

### Example 1 Preparation of HPR Inclusion Oily Stock Solution No.1

Each component according to the proportion of raw materials shown in Table 1 was weighed, and then follow the following steps:
(1) liposomal structural analog and emulsifier were provided, the mixture was stirred at 600 rpm for 2 hours, thereby mixed in a certain proportion to form an oil phase mixture;
(2) the oil phase mixture in step (1) was homogenized by stirring at 600 rpm (80-95 °C) for 30 minutes to form a mixture;
(3) then, the mixture was cooled down while stirring (such as cooled to 20-45 °C, preferably 20-40 °C);
(4) retinol or the derivative thereof was added, the mixture was stirred at 600 rpm, homogenized to form an inclusion mixture;
(5) subsequently, grease was added and thoroughly stirred to obtain HPR inclusion oily solution No.1 (100g in total).

**Table 1 Raw Material Formula (Percentage by Weight)**

| Raw Material | Examp le 1 | Examp le 2 | Examp le 3 | Examp le 4 | Examp le 5 | Examp le 6 | Comparat ive Example 1 | Comparat ive Example 2 | Comparat ive Example 3 |
|---|---|---|---|---|---|---|---|---|---|
| Hydroxypinacolone retinoate (HPR) | 10 | 10 | | 0.5 | 12 | 10 | 10 | 10 | |
| Retinol | | | 10 | | | | | | 10 |
| Liposomal Structural Analog | 1 | 9 | 1 | 1 | 1 | 1 | | | |
| Dicaprylyl Carbonate | 59 | 51 | 59 | 68.5 | 57 | 59 | 60 | | |
| Caprylic/Ca pric glycerides | 30 | 30 | 30 | 30 | 30 | 30 | 30 | | |
| Dimethyl Isosorbide (DMI) | | | | | | | | 90 | 90 |

### Example 2: Preparation of HPR Inclusion Oily Stock Solution No.2

Example 1 was repeated, with the difference being that the components were weighed according to the proportion of raw materials of Example 2 shown in Table 1, wherein the content of liposomal structural analog was increased to prepare HPR inclusion oily stock solution No.2.

### Example 3: Preparation of Retinol Inclusion Oily Stock Solution No.3

Example 1 was repeated, with the difference being that the components were weighed according to the proportion of raw materials of Example 3 shown in Table 1, wherein HPR was replaced with retinol to prepare retinol inclusion oily stock solution No.3.

### Example 4: Preparation of HPR Inclusion Oily Stock Solution No.4

Example 1 was repeated, with the difference being that the components were weighed according to the proportion of raw materials of Example 4 shown in Table 1, wherein the content of HPR was reduced to 0.5% to prepare HPR inclusion oily stock solution No.4.

### Example 5: Preparation of HPR Inclusion Oily Stock Solution No.5

Example 1 was repeated, with the difference being that the components were weighed according to the proportion of raw materials of Example 5 shown in Table 1, wherein the content of HPR was increased to 12% to prepare HPR inclusion oily stock solution No.5.

### Example 6: Preparation of Retinol Palmitate Inclusion Oily Stock Solution No.6

Example 1 was repeated, with the difference being that the components were weighed according to the proportion of raw materials of Example 6 shown in Table 1, wherein HPR was replaced with retinol palmitate to prepare retinol palmitate inclusion oily stock solution No.6.

### Comparative Example 1: Preparation of HPR Mixture Solution No.7

Example 1 was repeated, with the difference being that the components were weighed according to the proportion of raw materials of Comparative Example 1 shown in Table 1, wherein no liposomal structural analog was added to prepare HPR mixture solution No.7.

### Comparative Example 2: Preparation of HPR Mixture Solution No.8

Example 1 was repeated, with the difference being that the components were weighed according to the proportion of raw materials of Comparative Example 2 shown in Table 1, wherein the commonly used solvent for dissolving HPR, dimethyl isosorbide (DMI), was added as a positive control to prepare HPR mixture solution No.8.

### Comparative Example 3: Preparation of Retinol Mixture Solution No.8

Example 1 was repeated, with the difference being that the components were weighed according to the proportion of raw materials of Comparative Example 6 shown in Table 1, wherein the commonly used solvent for dissolving retinol, dimethyl isosorbide (DMI), was added as a positive control to prepare HPR mixture solution No.9.

### Test Example 1: Appearance Test

Test method: room temperature, take photos, and transparency was compared with the naked eye.

### Results:

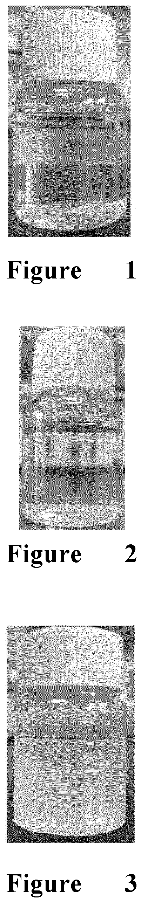

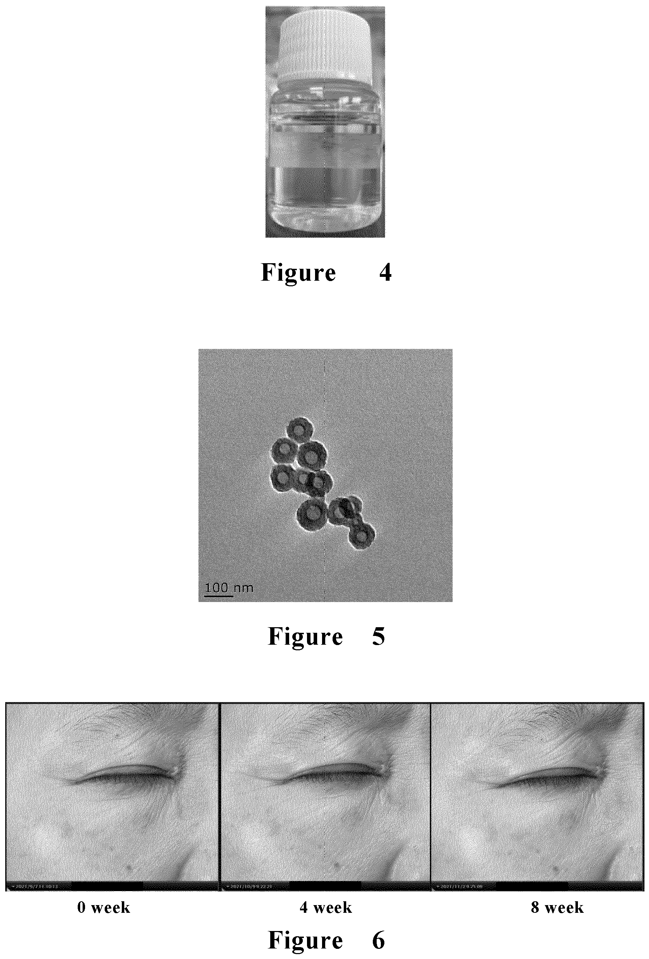

Example 1: transparent appearance (Figure 1);
Example 2: transparent appearance; the result is similar to Figure 1
Example 6: transparent appearance (Figure 2)
Comparative Example 1: cloudy appearance (Figure 3)
Comparative Example 2: transparent appearance (Figure 4).

The results of the above optical photos indicated that using liposomal structural analog of the present invention can effectively stabilize retinol and the analogues thereof that are difficult to stabilize with ordinary emulsifiers and oils, thereby obtaining a transparent oil solution; the mixture obtained using common solvent is also transparent.

### Test Example 2

The inclusion of Example 1 was characterized by transmission electron microscopy, and the result is shown in Figure 5. It can be seen that an internal hollow inclusion with an external shell layer was formed, with a size between 70-120 nm.

### Test Example 3: Stability Test

### 1) Appearance stability

Testing method: the samples were placed at room temperature for one month, for 45 °C cycle (24 hours at 45 °C, 24 hours at room temperature, and cycled for 1 month), for 5 °C cycle (24 hours at 45 °C, 24 hours at room temperature, and cycled for 1 month), for -10 °C cycle (24 hours at 45 °C, 24 hours at room temperature, and cycled for 1 month) for visual comparison of transparency; under light (irradiated under D65 standard light source, placed in a specified packaging material, irradiated for 8 hours per day for 30 consecutive days), the results are shown in Table 2:

**Table 2 Stability Test Results**

| | Example 1 | Example 2 | Example 3 | Example 4 | Example 5 | Example 6 | Comparative Example 1 | Comparative Example 2 | Comparative Example 3 |
|---|---|---|---|---|---|---|---|---|---|
| Room temperature (20 °C) 1 month | the appearance remains transparent | the appearance remains transparent | the appearance remains transparent | the appearance remains transparent | the appearance remains transparent | the appearance remains transparent | precipitation appears | the appearance is transparent | the appearance remains transparent |
| 45 °C, 1 month | the appearance remains transparent | the appearance remains transparent | the appearance remains transparent | the appearance remains transparent | the appearance remains transparent | the appearance remains transparent | precipitation appears | the appearance is transparent | the appearance remains transparent |
| 5 °C, 1 month | the appearance remains transparent | the appearance remains transparent | the appearance remains transparent | the appearance remains transparent | the appearance remains transparent | the appearance remains transparent | precipitation appears | the appearance is transparent | the appearance remains transparent |
| Light | the appearance is still transparent | the appearance is transparent | the appearance is transparent | the appearance is transparent | the appearance is transparent | the appearance is transparent | cloudy | the appearance is transparent | the appearance is transparent |

### 2) Content stability

HPR is a derivative of retinol, similar to retinol, which is unstable to light, heat, acid, and alkali and easily oxidized, but relatively more stable than retinol. In the application of cosmetic compositions, the stability of HPR under photothermal condition is particularly important.

This example selected HPR inclusion oily stock solution No.1 prepared in Example 1 (wherein mass percentage of HPR is 10%) and HPR mixture solution No.4 using dimethyl isosorbide as a solvent. The above samples were placed at 25 °C, 45 °C, and under light condition respectively for stability experiments, and were taken out at 1, 2, 3, and 4 weeks to measure the content of retinol using HPLC method to investigate the content stability. Acetonitrile: 2% acetic acid aqueous solution = 98:2 (v/v) was used as the mobile phase, carbon-18 reverse phase high performance liquid chromatography column (150mm × 4.6mm, 5 µm) was used as the chromatographic column , column temperature was 35 °C, flow rate was 1.0 mL/min, detection wavelength was 359 nm, and injection volume was 5 uL.

As shown in Table 3, the content of HPR in the sample of Example 1 changed very little and remained almost unchanged at 25 °C, 45 °C, and under light condition within 30 days. However, under the same conditions, the content of HPR in Comparative Examples 1 and 2 changed significantly. Under light condition, the content of HPR in Comparative Example 2 decreased from 10.0% to 8.8% within 30 days, while the effect of Comparative Example 1 was slightly better than that of Comparative Example 2 but still inferior to the stability of the highly dispersed inclusion oily stock solution in Example 1 due to its inability to disperse uniformly.

In addition, by comparing Example 3 and Comparative Example 3, HPR was replaced with retinol, in Comparative Example 3, the content of retinol decreased from 10.0% to 8.5% within 30 days, while the encapsulated oily stock solution in Example 3 showed better stability. The retinol group and HPR group obtained similar results, indicating that the encapsulated oily stock solution had a good protective effect on retinol and the derivative thereof.

It can be seen from Examples 4 and 5 that reducing and increasing the dosage of HPR can still maintain a relatively stable content within 30 days.

**Table 3 Results of Light Stability Test**

| **Experimental group /HPR content (%)** | **time/day** | | | |
|---|---|---|---|---|
| | **0** | **7** | **14** | **30** |
| **Example 1 (25 °C)** | **10.0** | **10.2** | **10.1** | **10.0** |
| **Example 1 (45°C)** | **10.0** | **10.1** | **9.8** | **9.9** |
| **Example 1 (Light)** | **10.0** | **9.9** | **9.9** | **9.8** |
| **Example 3 (Light)** | **10.0** | **9.8** | **9.7** | **9.7** |
| **Example 4 (Light)** | **0.5** | **0.48** | **0.48** | **0.47** |
| **Example 5 (Light)** | **12** | **11.9** | **11.8** | **11.8** |
| **Example 6 (Light)** | **10.0** | **9.9** | **9.8** | **9.8** |
| **Comparative Example 1 (Light)** | **10.0** | **9.6** | **9.2** | **9.0** |
| **Comparative Example 2 (25 °C)** | **10.0** | **10.0** | **9.5** | **9.5** |
| **Comparative Example 2 (45 °C)** | **10.0** | **9.8** | **9.5** | **9.4** |
| **Comparative Example 2 (Light)** | **10.0** | **9.5** | **9** | **8.8** |
| **Comparative Example 3 (Light)** | **10.0** | **9.3** | **8.8** | **8.5** |

### Test Example 4: Human Efficacy Experiment

In order to demonstrate the efficacy of the oily stock solution prepared by the present invention, the inclusion oily solution No.1 Example 1was added to the eye cream and subjected to human efficacy testing. The eye cream formula was shown in Table 4 below:

**Table 4 Eye Cream Formula**

| **Product Name** | | **Anti Wrinkle Eye Cream** | | |
|---|---|---|---|---|
| **Date** | | | | |
| **Serial Number** | **Phase** | **Item Name** | **Standard Name** | **Content** |
| 1 | A | Water | Water | 69.21 |
| 2 | | U-21 | Acrylates C10-30 Alkyl Acrylate Cross Polymer | 0.6 |
| 3 | | Acetyl Glucosamine | Acetyl Glucosamine | 2 |
| 4 | | Glycerol | Propanetriol | 3 |
| 5 | | 1,3-Butanediol | 1,3-Butanediol | 5 |
| 6 | | Allantoin | Allantoin | 0.15 |
| 7 | | Dipotassium Glycyrrhizinate | Dipotassium Glycyrrhizinate | 0.1 |
| 8 | | SymSaveTMH | Hydroxyacetophe | 0.3 |
| 9 | | Disodium EDTA | Disodium EDTA | 0.03 |
| 10 | B | Dow-9576 | | 3 |
| 11 | | OLI-7105 | | 3 |
| 12 | | VE | | 0.3 |
| 13 | | EMT-10 | Hydroxyethylacrylate / Sodium Acryloyldimethyl Taurate Copolymer | 1.2 |
| 14 | C | OLI-8109 | | 5 |
| 15 | | OLI-8100E | | 0.5 |
| 16 | D | Arginine | Arginine | 0.5 |
| 17 | | Water | Water | 3 |
| 20 | | OLI-1901 (inclusion oil stock solution) | | 2 |
| 21 | | P-21 | MENTHA ARVENSIS leaf extract/PELARGONIUM GRAVEOLENS extract/CHRYSANTHELLUM INDICUM extract/SOPHORAANGUSTIFOLIA root extract/PORTULACAOLERACEA extract/ALOE BARBADENSIS leaf extract/Butanediol/Glycerol/Sodium benzoate/Potassium sorbate/Water | 0.6 |
| 22 | | PE | Phenoxyethanol | 0.5 |
| 23 | | Flavor | SES015518 AWalk in the Woods | 0.01 |

Test site: Periocular region
Number of testers: 20 people
Test conditions: temperature 21 ± 1 °C, humidity 50 ± 10 %
Testing time: 30 subjects used anti wrinkle eye cream once in the morning and once in the evening per day, with self comparison before and after usage. The testing time points are: 0 week, 4 weeks, and 8 weeks.

Main experimental equipments: as shown in Table 5

**Table 5 Equipment for efficacy test evaluation**

| Name | Testing Index |
|---|---|
| Facial imaging system VISIA | Take pictures |
| Skin microscope and active skin surface analysis system Visioscan ^{®} VC 20plus | SEw-Skin wrinkles |

The test results of skin wrinkle area and proportion of wrinkle area were shown in Table 6. The wrinkle area value at week 0 is 78.97, after using the eye cream for 4 and 8 weeks, the wrinkle area value showed a stable and gradually decreasing trend, decreasing by 1.37% and 6.67% respectively. The proportion of area at week 0 was 24.24%, after using eye cream for 4 and 8 weeks, the proportion of area decreased by 1.46% and 6.86%, respectively.

**Table 6 Wrinkle Area and Wrinkle Proportion**

| **Experimental group/Wrinkle condition** | **Time/Week** | | |
|---|---|---|---|
| | **0** | **4** | **8** |
| **Wrinkle area** | **78.97** | **77.91** | **73.97** |
| **Proportion of wrinkle area (%)** | **24.24** | **23.89** | **22.68** |

VISA high-definition image comparison: Pre and post use images of volunteers were selected to compare wrinkles and textures around the eyes. From Figure 6, it can be seen that after using anti-aging eye cream for 8 weeks, the wrinkles at the end and under the eyes of the volunteers were significantly reduced or lightened.

All documents mentioned herein are incorporated by reference in this application as if each document were individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. An inclusion oily stock solution of retinol and a derivative thereof, comprising the following components in 100 parts by weight:
0.2-15 parts by weight of the retinol and the derivative thereof;
0.5-10 parts by weight of a liposomal structural analog;
10-50 parts by weight of an emulsifier; and
the balance of grease.

2. The inclusion oily stock solution according to claim 1, wherein the oily stock solution comprises the following components in 100 parts by weight:
0.5-12 parts by weight of the retinol and the derivative thereof;
1-10 parts by weight of a liposomal structural analog;
20-30 parts by weight of an emulsifier; and
the balance of grease.

3. The inclusion oily stock solution according to claim 1, wherein the retinol and the derivative thereof are selected from the group consisting of retinol, retinal, retinoic acid, retinol palmitate, hydrogenated retinol, retinol acetate, retinol linoleate, retinol propionate, retinol retinoate, hydroxypinacolone retinoate, and combinations thereof.

4. The inclusion oily stock solution according to claim 1, wherein the derivative of retinol is selected from the group consisting of hydroxypinacolone retinoate, retinol palmitate, and combinations thereof.

5. The inclusion oily stock solution according to claim 1, wherein the liposomal structural analog is a polymer containing:
(a) backbone unit A; and
(b) hydrophilic group L1 and optionally (c) hydrophobic group L2 located on the side chain or end of the polymer.

6. The inclusion oily stock solution according to claim 5, wherein the hydrophilic group L1 is represented by formula II: 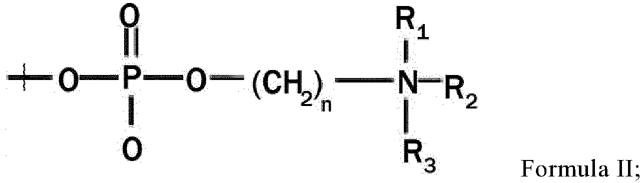
wherein, R₁, R₂, and R₃ are each independently selected from H, C₁-C₄ alkyl, C₂-C₄ alkenyl, and C₃-C₄ cycloalkyl;
n is an integer from 1 to 50 (e.g. 1, 2, 3, 4, 5, 10, or 15).

7. The inclusion oily stock solution according to claim 5, wherein the hydrophilic group L1 is located on the side chain, end or branch of the polymer.

8. The inclusion oily stock solution according to claim 5, wherein the backbone unit A is selected from the group consisting of:
A1) 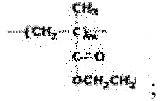 A2) 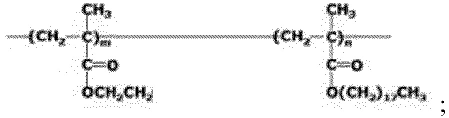
A3) 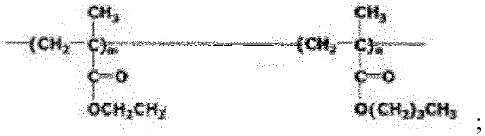
A4) 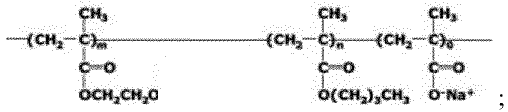 and
A5) combinations of A1-A4;
wherein, in A1 to A5, each m, n, and o is independently selected from an integer ranging from 1 to 1000 (2, 3, 5, 8, 10, 15, 20, 50, 100, 200, 300, 500, or 1000).

9. The inclusion oily stock solution according to claim 5, wherein the molecular weight of the liposomal structural analog is between 10,000 and 3 million, more preferably between 50,000 and 2 million.

10. The inclusion oily stock solution according to claim 1, wherein the retinol and the derivative thereof are encapsulated by the liposomal structural analog to form the inclusion.

11. The inclusion oily stock solution according to claim 1, wherein the grease is selected from the group consisting of dioctyl carbonate, octyldodecanol isostearate, isopropyl palmitate, isooctyl palmitate, isononyl isononanoate, white oil, caprylic/capric triglyceride, octyldodecanol, cocinic acid, lauryl alcohol, isopropyl myristate, shea butter oil, isooctyl 12-hydroxystearate, diisostearyl malate, squalane, jojoba oil, soybean oil, olive oil, sweet almond oil, avocado oil, wheat germ oil, and combinations thereof.

12. The inclusion oily stock solution according to claim 1, wherein the emulsifier is selected from the group consisting of polyglycerol esters, dehydrated sorbitol fatty acid esters, sorbitol cocoate, sucrose fatty acid esters, alkyl glycosides, hydrogenated soft phospholipids, fatty alcohol ethoxylates, alkylphenol ethoxylates, polyoxyethylene fatty acid, polyethers, caprylic/capric glycerides, and combinations thereof.

13. A method for preparing the inclusion oily stock solution according to claim 1, comprising the steps of:
(1) providing a liposomal structural analog and an emulsifier, and mixing them into an oil phase mixture;
(2) subjecting the oil phase mixture in step (1) to high-temperature homogenization and stirring to form a mixture;
(3) cooling down subsequently;
(4) adding retinol and the derivative thereof, stirring and homogenizing to form an inclusion mixture; and
(5) adding grease and stirring thoroughly to obtain the inclusion oily stock solution.

14. A cosmetic composition comprising the inclusion oily stock solution according to claim 1.

15. An inclusion of retinol and the derivative thereof, comprising the following components in parts by weight:
0.2-15 parts by weight of the retinol and the derivative thereof;
0.5-10 parts by weight of a liposomal structural analog; and
10-50 parts by weight of an emulsifier;
wherein, the retinol and the derivative thereof are encapsulated by liposomal structural analog, thereby forming the inclusion.
